# FASCICULE DE BREVET EUROPEEN

(11) **EP 3 297 525 B1**
(45) Date de publication et mention de la délivrance du brevet: **05.04.2023**
(21) Numéro de dépôt: 16733145.3
(22) Date de dépôt: 20.05.2016
(51) Int. Cl.: A61B 5/00, A61B 5/20, A61B 5/22, A61B 5/11

(54) **SONDE DE DIAGNOSTIC POUR MESURER LA DÉFORMATION D'UNE ENDOCAVITÉ ET LA RESISTANCE D'AU MOINS UN MUSCLE DU PÉRINÉE**
DIAGNOSTISCHE SONDE ZUR MESSUNG DER VERFORMUNG EINER ENDOKAVITÄT UND DES WIDERSTANDS VON MINDESTENS EINEM DAMMMUSKEL
DIAGNOSTIC PROBE FOR MEASURING THE DEFORMATION OF AN ENDOCAVITY AND THE RESISTANCE OF AT LEAST ONE PERINEAL MUSCLE

(30) Priorité: 21.05.2015 FR 1501063
(43) Date de publication de la demande: 28.03.2018
(73) Titulaire: Akse, 77550 Moissy-Cramayel (FR)
(72) Inventeur: BILLARD, Georges, 34470 Perols (FR)
(74) Mandataire: Rhein, Alain
(86) Numéro de dépôt international: PCT/FR2016/051200
(87) Numéro de publication internationale: WO 2016/185147

(56) Documents cités:
- EP-A1- 1 600 103
- WO-A1-2013/116310
- US-A1- 2004 030 360
- US-A1- 2007 293 792
- US-A1- 2015 112 230

## Description

La présente invention concerne le domaine paramédical et médical et, plus particulièrement, l'analyse de l'état fonctionnel des structures anatomiques impliquées dans le mécanisme de la continence et de l'incontinence urinaire et/ou fécale.

Par exemple, mais non limitativement, cette analyse peut être effectuée en pré ou post-partum pour mettre en évidence les conséquences fonctionnelles d'un traumatisme obstétrical.

En effet, après un tel traumatisme, notamment un accouchement par voies naturelles, certaines pathologies périnéales et/ou sphinctériennes peuvent se développer et engendrer des troubles notamment urinaires, ano-rectaux et/ou sexuels qui peuvent s'avérer gênants, voire handicapants, pour la patiente.

De ce fait, il est nécessaire, dans un premier temps, de procéder à un diagnostic de l'état fonctionnel des muscles du périnée de la patiente (ou du patient) afin de lui proposer, dans un second temps, des séances de rééducation adaptées et spécifiques ou un traitement chirurgical en fonction des résultats qui ont été préalablement obtenus.

De manière générale, l'incontinence est due à une augmentation de la pression intra-abdominale à laquelle les muscles de clôture de l'urètre et/ou du canal anal fournissent une réponse trop lente et/ou trop faible et/ou mal coordonnée.

Or, les muscles de clôture de l'urètre et/ou du canal anal comportent plusieurs groupes de muscles.

Un premier groupe de muscles regroupe les sphincters urétraux et anaux qui sont des muscles qui ceinturent et verrouillent respectivement, en se contractant, l'urètre et le canal anal.

Un deuxième groupe de muscles regroupe les muscles pubo-vaginaux et pubo-rectaux qui cravatent le vagin et le canal anal, ces muscles participent à la clôture de l'urètre et du canal anal en réalisant une déformation mécanique antéro-postérieure du vagin et du canal anal .

Le troisième groupe de muscles regroupe les muscles du périnée en forme de coupole et qui entourent l'extrémité inférieure du vagin et du canal anal, les muscles du périnée participent au verrouillage de l'urètre et/ou du canal anal en amortissant, par déformation, l'augmentation de la pression intra-abdominale . En outre, les muscles du périnée soutiennent les organes qui se trouvent dans l'abdomen, par exemple, la vessie, l'urètre, le vagin et l'utérus pour une femme, la prostate pour un homme et le rectum.

D'autres facteurs participent également à l'amortissement de l'augmentation de la pression intra-abdominale, comme la masse de graisse contenue dans la fosse ischio-rectale ainsi que les ligaments maintenant les organes à l'intérieur de la cavité abdominale.

Il est à noter que ces ligaments présentent une faible capacité de déformation. Ainsi, lors d'une déformation mécanique, leur résistance est d'autant plus importante que la force et la vitesse de la déformation mécanique qui leur est appliquée sont importantes .

Actuellement, afin de procéder à une analyse fonctionnelle des structures anatomiques liées au mécanisme de l'incontinence, un praticien, par exemple un médecin ou un physiothérapeute, réalise une analyse fonctionnelle, de façon manuelle, du seul périnée d'une patiente.

Le praticien doit alors ressentir le résultat de la contraction du périnée qui est initiée par la patiente, en apprécier la qualité, puis verbaliser la commande, entendue par la patiente, afin qu'elle modifie en réponse la contraction de son périnée.

De telles pratiques n'offrent malheureusement aucune régularité, car elles sont très dépendantes du praticien et de son expérience et sont limitées à l'analyse fonctionnelle du périnée qui n'est qu'une des structures anatomiques impliquées dans le mécanisme de continence et d'incontinence.

Afin de tenter de remédier aux inconvénients des méthodes manuelles, le document de brevet FR 2 941 860, déposé par la demanderesse elle-même, décrit un instrument de mesure des différents paramètres fonctionnels et organiques du plancher pelvien, c'est-à-dire des muscles du périnée.

Cet instrument comprend au moins deux branches articulées en rotation de manière à passer d'une position fermée à une position ouverte, et inversement, reproduisant ainsi les doigts du praticien, ledit instrument comprenant en outre des moyens de contrôle magnétique de l'angle d'écartement des branches.

Dans un souci de diagnostic, l'instrument décrit dans ce document permet de déterminer l'origine des dysfonctionnements affectant les muscles périnéo-sphinctériens en mettant en évidence, notamment, un déficit fonctionnel des différents types de fibres musculaires (toniques et phasiques) , un retard de déclenchement du réflexe myotatique de verrouillage périnéal, une fibrose du tissu musculaire, une atteinte neurologique, ou encore une distension du tissu conjonctif. Cependant, bien que très performant, et amenant déjà une amélioration substantielle par rapport à ce qui préexistait dans l'état de la technique, ce dispositif peut encore être perfectionné.

En particulier, face à la complexité du mécanisme de l'incontinence décrit ci-dessus, ledit dispositif n'offre pas la possibilité de mesurer certains paramètres comme la déformation et le déplacement de la cavité vaginale et/ou rectale due à l'augmentation de pression intra-abdominale et/ou à la réponse des muscles pubo-vaginaux et/ou pubo-rectaux.

Or la mesure de tous les paramètres impliqués dans le mécanisme de l'incontinence permettrait un diagnostic précis de la cause de l'incontinence et en particulier de l'une des principales causes qu'est l'hypermobilité urétrale et périnéale. Cette mesure permettrait notamment de définir précisément à quel niveau de déplacement des organes et à quel niveau de pression intra-abdominale se produisent les fuites urinaires ou anales.

Dans l'état de l'art, l'IRM permet de mesurer le déplacement des organes mais ne permet pas d'établir une corrélation entre les pressions, les déplacements et la fuite. Or, l'établissement d'un diagnostic précis permettrait de parfaitement définir les indications de chirurgie ou de rééducation et d'optimiser les exercices qui sont proposés ensuite à la patiente, ou au patient, dans l'optique d'une rééducation de la fonction périnéale.

Le document US 2004/030360A1 divulgue un dispositif intravaginal pour stimuler électriquement les muscles et les nerfs définissant et entourant la cavité intravaginale et/ou pour détecter l'activité électrique desdits muscles, et, plus particulièrement, un dispositif et un système l'utilisant qui sont utiles pour prévenir ou traiter dysfonctionnement du plancher pelvien chez la femme. Le dispositif comporte un corps construit de sorte que lorsqu'il est contracté et positionné à l'intérieur d'une cavité intravaginale de l'individu, le corps se dilate automatiquement pour se conformer à un contour de la cavité intravaginale. Le dispositif comprend en outre au moins une paire d'électrodes servant à stimuler électriquement les muscles et/ou les nerfs définissant et entourant la cavité intravaginale. Il comprend, par ailleurs, au moins un capteur pour mesurer l'activité des muscles de la cavité intravaginale disposé sur le corps flexible, le capteur étant soit un capteur de pression, soit un capteur d'activité électrique.

L'invention offre ainsi la possibilité de pallier les divers inconvénients de l'état de la technique en proposant une sonde permettant de mesurer, en réponse à une augmentation de pression intra-abdominale, le déplacement et la déformation mécanique du périnée et de la cavité vaginale ou rectale induite, par exemple, par une toux ainsi que le comportement des muscles de clôture de l'urètre ou de la cavité anale et le point de départ d'une fuite urinaire par rapport aux niveaux de pression et de déformation.

A cet effet, la présente invention concerne une sonde de diagnostic telle que définie par la revendication 1.

Selon un mode de réalisation préféré de l'invention , la sonde de diagnostic coopère avec des moyens d'analyse des mesures de pression/déplacement effectuées et de reconstitution d'une vision tridimensionnelle dynamique du déplacement de ladite endocavité.

Cette caractéristique permet de modéliser avec précision le déplacement et la déformation de l'endocavité suite à une augmentation de la pression intra-abdominale et ainsi de se substituer à un examen coûteux et beaucoup moins complet qui serait réalisé à l'aide d'une imagerie par résonance magnétique ou « IRM ».

Selon une particularité de l'invention, le corps endocavitaire est formé par une bande souple pliée en deux de manière à épouser, dans un plan sagittal, les parois de ladite endocavité, le corps endocavitaire comportant deux extrémités chacune reliée à l' embase ex vivo de la sonde de diagnostic, l'embase ex vivo comportant un moyen adhésif biocompatible pour la renforcement du maintien en position de ladite sonde.

La souplesse du corps endocavitaire permet d'éviter, que lors d'un examen, la sonde de diagnostic ne soit expulsée de l'endocavité par une descente d'organe ou « prolapsus » d'un des organes entourant l'endocavité dans laquelle la sonde est insérée.

En outre, afin de déterminer à quel moment se produit la fuite urinaire suite à une augmentation de la pression intra-abdominale, l'embase ex vivo comporte au moins un détecteur d'urine.

Selon un mode de réalisation particulier de l'invention, le moyen de mesure du déplacement de la paroi endocavitaire consiste en une centrale inertielle.

Afin d'obtenir des mesures précises du déplacement et de la déformation de l'endocavité dans laquelle la sonde de diagnostic est insérée, l'élément de référence spatial X,Y,Z comporte au moins deux moyens de mesure du déplacement du centre tendineux du périnée et du sacrum.

Selon une caractéristique additionnelle de l'invention, la sonde de diagnostic coopère avec au moins deux électrodes de mesure d'un signal électro-myographique disposées sur l'abdomen d'un ou d'une patient(e) de manière à enregistrer l'activité électromyographique abdominale à l'origine des pressions intra-abdominales.

La coopération entre la sonde de diagnostic selon l'invention et les électrodes électromyographiques permet de réaliser un suivi des déformations et déplacements de l'endocavité dans laquelle la sonde de diagnostic est insérée tout au long de l'examen selon une échelle de temps. Ainsi, il est possible de déterminer précisément à quel moment se produit la fuite urinaire et quelle structure anatomique en est responsable.

Afin de mesurer le déplacement et la déformation des parois de l'endocavité à différents niveaux de profondeur, le corps endocavitaire comporte quatre moyens de mesure du déplacement de la paroi endocavitaire.

Cette caractéristique permet de mesurer, de manière indirecte, le déplacement et la déformation des structures anatomiques qui entourent l'endocavité dans laquelle la sonde de diagnostic est insérée.

De la même manière, afin de mesurer à quel niveau de résistance se produit la fuite urinaire, le corps endocavitaire comporte six capteurs de pression. Ces capteurs de pression sont répartis par paire le long du corps endocavitaire, et permettent de mesurer les pressions antéro-postérieures qui sont appliquées aux parois de l'endocavité à trois niveaux différents de profondeur.

Pour des raisons d'hygiène, lorsque la sonde est insérée dans une endocavité, elle coopère avec une enveloppe en matériau polymérique biocompatible recouvrant le corps endocavitaire.

La présente invention comporte de nombreux avantages. D'une part, la sonde de l'invention permet une mesure, selon les 3 axes X, Y et Z, des déformations et de la résistance passive et active des structures anatomiques responsables de la continence et de l'incontinence. D'autre part, en corrélation avec la pression antéro-postérieure que subit l'endocavité dans laquelle la sonde est insérée, le moment où la fuite se produit, et le début de l'augmentation de la pression intra-abdominale, il est possible d'identifier de façon précise à quel moment se déroule la fuite, à quel niveau de résistance se déroule la fuite et quelle structure anatomique est à l'origine de l'incontinence.

En outre, au moyen de la présente sonde, le diagnostic peut être établi dans les conditions physiologiques dans lesquelles sont susceptibles de se manifester les désagréments liés aux troubles de la continence, c'est-à-dire en position verticale, lorsque le patient est debout. Les désagréments tels qu'évoqués peuvent notamment consister en des fuites urinaires.

Un autre avantage de l'invention réside dans le fait que la présente sonde permet l'établissement d'un diagnostic de manière indépendante de l'opérateur qui manie ladite sonde. De ce fait, quelle que soit l'expérience du praticien, le diagnostic établit sera extrêmement fiable.

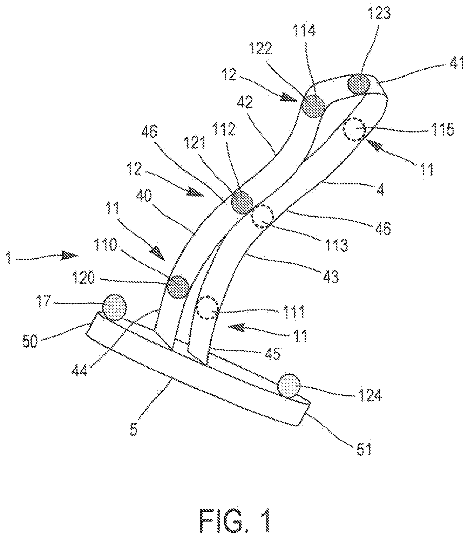

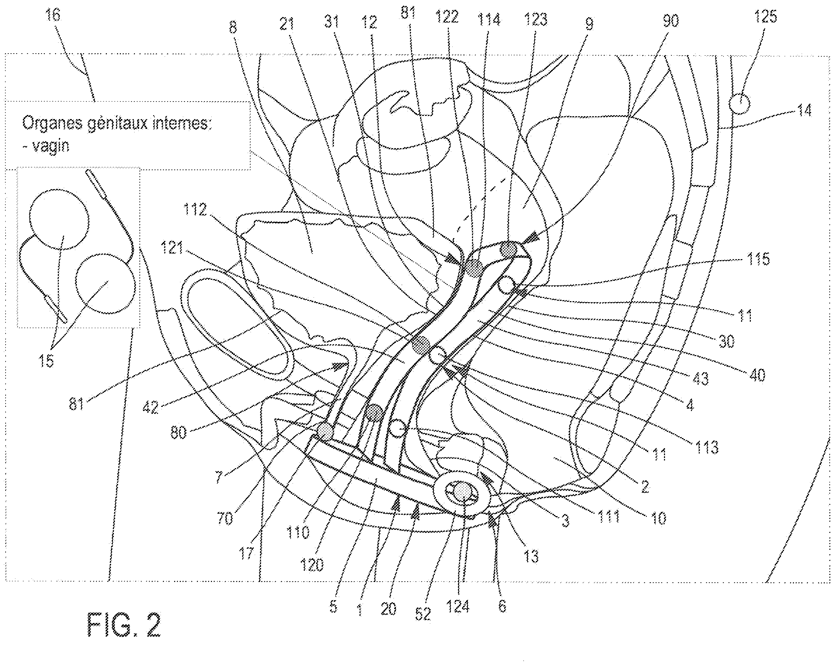

D'autres caractéristiques et avantages de l'invention ressortiront de la description détaillée qui va suivre d'un exemple de réalisation non limitatif de l'invention, en référence aux figures 1 et 2 annexées dans lesquelles :
- La figure 1 est une vue en perspective d'une sonde de diagnostic conforme à un mode de réalisation de l'invention ; et
- La figure 2 est une vue de la sonde de diagnostic de la figure 1, la sonde de diagnostic étant insérée dans une cavité endovaginale.

Tel que représentée sur la figure 1, la présente invention concerne une sonde de diagnostic 1 endocavitaire, autrement dit destinée à être insérée dans une cavité corporelle d'un patient, plus souvent d'une patiente.

La sonde de diagnostic 1 selon l'invention peut ainsi être introduite dans la cavité endovaginale 2 d'une patiente, dans ce cas il s'agit d'une sonde « endovaginale », ou bien dans la cavité rectale et, dans ce cas, c'est une sonde dite « endorectale ». Dans la suite de la description détaillée, le terme endocavité désigne indifféremment une cavité endovaginale 2 et une cavité endorectale.

Dans l'exemple de réalisation illustré aux figures 1 et 2, la sonde de diagnostic 1 est insérée dans une cavité endovaginale 2 et permet de mesurer le déplacement et la déformation mécanique des parois 3 de la cavité endovaginale 2.

Afin d'éviter de modifier le déplacement et/ou la déformation mécanique des parois 3 de l'endocavité, la sonde de diagnostic 1 comporte un corps endocavitaire 4 souple déformable qui ne présente pas de résistance à la déformation.

Avantageusement, une fois insérée dans l'endocavité, par exemple à l'aide d'une pince gynécologique, le corps endocavitaire 4 épouse parfaitement les parois 3 de l'endocavité.

Dans cette optique, le corps endocavitaire 4 est formé par une bande souple 40 pliée en deux de manière à former une courbe sommitale 41 qui épouse, dans un plan sagittal, les parois 3 de ladite endocavité.

L'expression « plan sagittal » est utilisée pour désigner le plan médian qui sépare la moitié droite de la moitié gauche du corps humain. En réalité, le plan médian est l'un des innombrables plans sagittaux, ceux-ci étant tous parallèles audits plans médians.

Ainsi, dans le présent exemple on peut qualifier la partie de la bande souple 40 qui est en contact avec la paroi endocavitaire antérieure 30 de l'endocavité, de partie antérieure 42 du corps endocavitaire 4. De la même manière, on peut qualifier la partie de la bande souple 40 en contact avec la paroi endocavitaire postérieure 31 de l'endocavité, de partie postérieure 43 du corps endocavitaire. Les parties antérieure 42 et postérieure 43 de la bande souple 40 sont reliées entre elles supérieurement par la courbe sommitale 41 de la bande souple 40.

De préférence, cette bande souple 40 est constituée d'un circuit imprimé en polycarbonate flexible. Avantageusement, l'utilisation d'une bande souple 40 en polycarbonate permet d'équiper le corps endocavitaire 4 de dispositifs électroniques tels que des capteurs de données physico-chimiques.

Comme illustré aux figures 1 et 2, le corps endocavitaire 4 comporte deux extrémités, une extrémité antérieure 44 reliée à une partie antérieure 50 d'une embase 5 ex vivo, et une extrémité postérieure 45 reliée à une partie postérieure 51 de l'embase 5. Ainsi, lorsque la sonde diagnostic 1 est insérée dans une endocavité, l'embase 5 est disposée à l'ouverture 20 de l'endocavité. Ici, la sonde de diagnostic 1 est insérée dans la cavité endovaginale 2 d'une patiente, l'embase 5 étant disposée à la commissure des grandes lèvres (illustré à la figure 2).

Avantageusement, l'embase 5 comporte un détrompeur permettant d'insérer la sonde de diagnostic 1 dans l'endocavité dans le plan sagittal de manière à ce que la partie antérieure 42 du corps endocavitaire 4 soit disposée dans un plan sagittal contre la paroi antérieure 30 de l'endocavité, et à contrario que la partie postérieure 43 du corps endocavitaire 4 soit disposée contre la paroi postérieure 31 de l'endocavité toujours dans un plan sagittal.

De plus, l'embase 5 comporte un moyen adhésif 52 biocompatible venant se fixer à la peau du/de la patient(e) de manière à sécuriser le maintien de la sonde de diagnostic 1 en position à l'intérieur de l'endocavité. De préférence, le moyen adhésif 52 biocompatible est positionné au niveau de l'espace recto-génital 6.

Comme indiqué précédemment, la sonde de diagnostic 1 de l'invention a pour objectif de mesurer, en réponse à une augmentation de pression intra-abdominale, le déplacement et la déformation mécanique de l'endocavité et des organes qui l'entourent, mais aussi le déplacement et la déformation des muscles du périnée. Avantageusement, ces mesures permettent de diagnostiquer de manière précise et fiable les causes de l'incontinence urinaire ou fécale.

En effet, suite, par exemple, à une grossesse, un accouchement, des changements hormonaux, ou encore au vieillissement, des dysfonctionnements peuvent apparaître par exemple au niveau des muscles du plancher pelvien, c'est-à-dire des muscles du périnée. Les muscles du périnée servent de support à de nombreux organes, incluant notamment l'urètre 7, la vessie 8, le vagin 21 et l'utérus 9 chez la femme, ou encore le rectum 10.

Toutefois, les muscles du périnée ne sont pas les seules structures anatomiques qui peuvent être responsables de l'incontinence urinaire ou fécale. Comme indiqué précédemment, l'incontinence peut être également due à un dysfonctionnement des muscles sphincters urétraux ou rectaux, des muscles pubo-vaginaux ou pubo-rectaux ou encore à une faiblesse ligamentaire provoquant une descente d'organe appelée « prolapsus », consécutive à une augmentation de la pression intra-abdominale. En effet, une incontinence urinaire ou fécale peut être due à une faiblesse ligamentaire causant un prolapsus de l'urètre 7, de l'utérus 9, du vagin 21, du rectum 10, ou encore de la vessie 8.

En outre, l'incontinence fécale ou urinaire se définit par une perte involontaire d'urine via l'urètre 7, suite à un effort physique (toux, éternuement, rire, etc.), et sans sensation de besoin ressentie au préalable.

Ainsi un diagnostic des troubles de l'incontinence, établi de manière fiable, permet par la suite, de proposer une rééducation et/ou un traitement optimisé(es) répondant aux besoins spécifiques du patient atteint de troubles de l'incontinence.

Comme illustré aux figures 1 et 2, afin d'identifier la ou les structures anatomiques responsables des troubles de l'incontinence d'un(e) patient(e), ladite sonde de diagnostic 1 comporte au moins deux capteurs 11 de pressions disposés sur le corps endocavitaire 4 et adaptés à mesurer les pressions au niveau des parois 3 de l'endocavité, ici la cavité endovaginale 2.

Selon un exemple de réalisation, ces capteurs Il de pression sont des capteurs piézo-électriques. Toutefois, lesdits capteurs 11 de pression de la sonde de diagnostic 1 peuvent être de tout type, à partir du moment où ils sont aptes à permettre de mesurer une force de pression.

Lesdits capteurs 11 de pression sont situés dans un même plan, et agissent tous soit dans une direction identique, soit dans deux directions opposées.

A cet effet, dans le présent exemple, les capteurs 11 de pression sont disposés respectivement sur la partie antérieure 42 et postérieure 43 du corps endocavitaire 4. Du fait de leur disposition sur le corps endocavitaire 4 de la sonde 1, une fois que le corps endocavitaire 4 de la sonde 1 est inséré dans l'endocavité du patient ou de la patiente, les capteurs 11 de pression sont positionnés dans un plan sagittal.

De préférence, la sonde de diagnostic 1 de l'invention comporte un nombre supérieur à deux capteurs 11 de pression, et, plus préférentiellement encore, ladite sonde 1 est équipée de six capteurs 11 de pression.

Comme illustré sur les figures jointes, les capteurs de pression 11 sont disposés deux à deux, par paire, dans un même plan, un premier capteur 11 de pression étant disposé sur la partie antérieure 42 du corps endocavitaire 4 et un second capteur 11 de pression sur la partie postérieure 43 du corps endocavitaire 4. De préférence, chaque couple de capteurs 11 de pression agit dans des directions opposées.

De cette manière, chaque paire de capteurs 11 de pression est disposée à différents niveaux sur le corps endocavitaire 4. Ainsi, lors de l'insertion du corps endocavitaire 4 dans une endocavité, ici la cavité endovaginale 2, chaque paire de capteurs 11 de pression se trouve positionnée à différents niveaux de profondeur dans la cavité endovaginale 2. En conséquence, il est possible de mesurer les pressions antéro-postérieures qui s'exercent en plusieurs niveaux de l'endocavité.

Comme illustré aux figures 1 et 2, le corps endocavitaire 4 comporte une première paire de capteurs 11 de pression, dont chaque capteur 11 est disposé de manière proximale à l'embase 5. La première paire de capteurs 11 de pression se compose d'un capteur antérieur 110 proximal de l'embase 5 disposé sur la partie antérieure 42 du corps endocavitaire 4 et d'un capteur postérieur 111 proximal de l'embase 5 disposé sur la partie postérieure 43 du corps endocavitaire 4.

Dans l'exemple illustré à la figure 2, lorsque le corps endocavitaire 4 est inséré dans une cavité endovaginale 2, les capteurs antérieur 110 proximal et postérieur 111 proximal de l'embase 5 se situent au niveau où la paroi 3 de la cavité endovaginale 2 est en contact avec les muscles pubo-vaginaux et pubo-rectaux. Ainsi, la première paire de capteurs 11 de pression permet, en réponse à une augmentation de la pression intra-abdominale, de mesurer indirectement la pression antéro-postérieure qu'exercent les muscles pubo-vaginaux et pubo-rectaux sur les parois 3 de la cavité endovaginale 2.

Le corps endocavitaire 4 comporte une deuxième paire de capteurs 11 de pression disposée à une position intermédiaire 46 entre l'embase 5 et la boucle sommitale 41 de la bande souple 40.

La deuxième paire de capteurs 11 de pression se compose d'un capteur antérieur 112 intermédiaire disposé sur la partie antérieure 42 du corps endocavitaire 4 et d'un capteur postérieur 113 intermédiaire disposé sur la partie postérieure 43 du corps endocavitaire 4.

Dans l'exemple illustré à la figure 2, une fois le corps endocavitaire 4 inséré dans la cavité endovaginale 2, le capteur de pression antérieur 112 intermédiaire se trouve dans une zone où la paroi 3 de la cavité endovaginale 2 est en contact avec la jonction du col vésical (JUV) 80, alors que le capteur de pression postérieur 113 intermédiaire se trouve une zone où la paroi 3 de la cavité endovaginale 2 est en contact avec le rectum 10.

Ainsi, la deuxième paire de capteurs de pression 11 permet de mesurer les pressions antéro-postérieures qu'appliquent le col vésical 80 et le rectum 10 au niveau de la paroi 3 de la cavité endovaginale 2.

Le corps endocavitaire 4 comporte une troisième paire de capteurs 11 de pression disposée sur la bande souple 40 de manière proximale à la courbe sommitale 41 de la bande souple 40.

La troisième paire de capteurs 11 de pression se compose d'un capteur antérieur 114 proximal sommital disposé sur la partie antérieure 42 du corps endocavitaire 4 et d'un capteur postérieur 115 proximal sommital disposé sur la partie postérieure 43 du corps endocavitaire 4.

Comme illustré à la figure 2, lorsque le corps endocavitaire 4 est inséré dans la cavité endovaginale 2, le capteur de pression antérieur 114 proximal sommital est situé dans une zone où la paroi 3 de la cavité endovaginale 2 est en contact avec le dôme vésical 81, c'est-à-dire, de la partie supérieure de la vessie 8. A contrario, une fois le corps endocavitaire 4 inséré dans la cavité endovaginale 2, le capteur de pression postérieur 115 proximal sommital se situe dans une zone où la paroi 3 de la cavité endovaginale 2 est en contact avec le rectum 10. Ainsi, la troisième paire de capteur de pression 11 permet de mesurer la pression antéro-postérieure qu'exercent le dôme vésical 81 et le rectum 10 sur la paroi 3 de la cavité endovaginale 2.

De plus, afin de mesurer le déplacement et la déformation de l'endocavité et des organes qui l'entourent, mais aussi le déplacement et la déformation des muscles du périnée, le tout suite à une augmentation de la pression intra-abdominale, la sonde de diagnostic 1 comporte également au moins un moyen de mesure 12 du déplacement des structures anatomiques, dans les trois axes X, Y et Z, soit une mesure du déplacement et de la déformation des structures anatomiques en trois dimensions ou « 3D ».

Dans l'exemple illustré aux figures 1 et 2, la sonde comporte au moins quatre moyens de mesure 12 du déplacement des structures anatomiques, qui sont disposés à différents niveaux de profondeur lorsque la sonde 1 est insérée dans une endocavité.

Les moyens de mesure du déplacement en 3D de la paroi 3 endocavitaire peuvent être formés par une centrale inertielle.

Plus précisément, une centrale inertielle correspond à un dispositif incorporant à la fois des capteurs d'accélération et de vitesse angulaire, par exemple trois gyromètres et trois accéléromètres. Ce dispositif de centrale inertielle permet un calcul, en temps réel, à partir des composantes de l'accélération, de l'évolution du vecteur vitesse et de la position de l'ensemble, ici la sonde diagnostic 1 de l'invention, au niveau duquel ladite centrale inertielle est positionnée.

Toutefois, les moyens de mesure 12 du déplacement peuvent être également formés par tout autre moyen adapté à la réalisation d'une telle mesure de déplacement et d'accélération dans les trois axes X, Y, Z.

Ainsi, afin de mesurer le déplacement des structures anatomiques qui entourent l'endocavité dans laquelle est insérée la sonde 1, le corps endocavitaire 4 est équipé de quatre moyens 12 de mesure de déplacement adaptés à mesurer le déplacement des parois 3 de l'endocavité à quatre différents niveaux de profondeur.

A cet effet, le corps endocavitaire 4 comporte un premier moyen de mesure 120 de déplacement disposé sur la bande souple 40 de manière proximale à l'embase 5. Dans le présent exemple, une fois le corps endocavitaire 4 inséré dans la cavité endovaginale 2, le premier moyen de mesure 120 de déplacement se situe au niveau où la paroi 3 de la cavité endovaginale 2 est en contact avec les muscles pubo-vaginaux et pubo-rectaux (illustré figure 2). Ainsi, le premier moyen de mesure 120 de déplacement permet, en réponse à une augmentation de la pression intra-abdominale, de mesurer indirectement le déplacement et la déformation des muscles pubo-vaginaux et pubo-rectaux au travers de la mesure du déplacement et de la déformation de la paroi 3 de la cavité endovaginale 2.

Le corps endocavitaire 4 comporte un deuxième moyen de mesure 121 du déplacement disposé dans une position intermédiaire entre l'embase 5 et la boucle sommitale 41 de la bande de souple 40. Ici, lorsque le corps endocavitaire 4 est inséré dans la cavité endovaginale 2, le deuxième moyen de mesure 121 de déplacement se situe au niveau où la paroi 3 de la cavité endovaginale 2 est en contact avec la jonction du col vésical 80 (illustré figure 2). Ainsi, le deuxième moyen de mesure 121 de déplacement permet, en réponse à une augmentation de la pression intra-abdominale, de mesurer indirectement le déplacement et la déformation de la jonction du col vésical 80 au travers de la mesure du déplacement et de la déformation de la paroi 3 de la cavité endovaginale 2.

Le corps endocavitaire 4 comporte un troisième moyen de mesure 122 de déplacement disposé sur la bande souple 40 à proximité de la courbe sommitale 41. Ainsi, lorsque le corps endocavitaire 4 est inséré dans une cavité endovaginale 2, le troisième moyen de mesure 122 de déplacement se situe au niveau où la paroi 3 de la cavité endovaginale 2 est en contact avec le dôme vésical 81 (illustré figure 2). Dès lors, le troisième moyen de mesure 122 du déplacement permet, en réponse à une augmentation de la pression intra-abdominale, de mesurer indirectement le déplacement et la déformation du dôme vésical 81 au travers de la mesure du déplacement et de la déformation de la paroi 3 de la cavité endovaginale 2. Ce troisième moyen de mesure 122 de déplacement permet entre autre d'identifier un prolapsus de la vessie 8.

Le corps endocavitaire 4 comporte un quatrième moyen de mesure 123 de déplacement disposé au sommet de la courbe sommitale 41 de la bande souple 40. Ici, lorsque le corps endocavitaire 4 est inséré dans la cavité endovaginale 2, le quatrième moyen de mesure 123 du déplacement se situe au niveau d'une zone de la paroi 3 de la cavité endovaginale 2 qui est en contact avec le col 90 de l'utérus 9 (illustré figure 2). Dès lors, le quatrième moyen de mesure 123 du déplacement permet, en réponse à une augmentation de la pression intra-abdominale, de mesurer indirectement le déplacement et la déformation du col 90 de l'utérus 9 au travers de la mesure du déplacement et de la déformation de la paroi 3 de la cavité endovaginale 2. Ainsi, ce quatrième moyen de mesure 123 du déplacement, permet notamment de mesurer et d'identifier un prolapsus de l'utérus 9.

En outre, selon une particularité du présent mode de réalisation illustré aux figures 1 et 2, les premier 120, deuxième 121 et troisième 122 moyens de mesure de déplacement sont disposés, d'une part, sur la partie antérieure 42 du corps endocavitaire 4, et d'autre part, respectivement à proximité immédiate des capteurs 110, 112, 114 de pression .

Etant donné que la sonde de diagnostic 1 est introduite au niveau d'une endocavité, les moyens de mesure 12 de déplacement permettent, de ce fait, une mesure du déplacement et de la déformation de la paroi 3 endocavitaire, et donc une mesure indirecte du déplacement et de la déformation des structures anatomiques qui entourent cette endocavité, notamment muscles pubo-vaginaux/pubo-rectaux, la vessie 8, les sphincters situés au niveau de la jonction du col vésical 80, la prostate/ou l'utérus 9 etc.

En outre, afin de mesurer le déplacement des muscles du périnée en réponse à une augmentation de la pression intra-abdominale, la sonde de diagnostic 1 comporte un cinquième moyen de mesure 124 de déplacement qui est disposé sur l'embase 5 de manière à être plaqué contre les centres tendineux 13 du périnée au niveau de l'anneau recto-génital. De cette manière, il est envisageable d'évaluer le déplacement des muscles du périnée dans toutes les directions, autrement dit du haut vers le bas, de l'avant vers l'arrière, et latéralement.

Avantageusement, dans le présent exemple, le moyen adhésif 52 biocompatible est disposé autour du cinquième moyen de mesure 124 de déplacement. Cette caractéristique permet d'assurer un contact permanent entre le cinquième moyen de mesure 124 du déplacement et l'anneau recto-génital. De plus, ce cinquième moyen de mesure 124 de déplacement constitue également un élément de référence spatial X,Y,Z aux moyens de mesure 120, 121, 122, 123 de déplacement qui se trouvent sur le corps endocavitaire 4.

Dans l'exemple illustré à la figure 2, afin d'enregistrer les mouvements globaux du bassin lors d'une augmentation de la pression intra-abdominale et ainsi de constituer en cas de mouvement du corps du patient ou de la patiente un élément référence spatial X,Y,Z, aux autres moyens de mesure 120, 121, 122, 123, 124 du déplacement, la sonde de diagnostic 1 est reliée à un sixième moyen de mesure 125 de déplacement.

Ce sixième moyen de mesure 125 de déplacement est positionné sur le corps du patient ou de la patiente, de préférence au niveau du sacrum 14. Ce sixième moyen de mesure 125 de déplacement permet d'éviter, en cas de mouvement intempestif ou volontaire, effectué par le (la) patient(e) lors de l'examen, de fausser les mesures de déplacement de la paroi 3 de la cavité endovaginale 2, des organes entourant le vagin 21 et des muscles du périnée. En effet, afin de réaliser un diagnostic de l'incontinence, il sera généralement demandé au patient ou à la patiente, par exemple, de tousser, et cela dans l'optique d'obtenir une mesure dans une situation d'effort physique qui est susceptible d'aboutir, notamment, à une incontinence urinaire d'effort.

De manière plus précise, l'incontinence urinaire d'effort, qui correspond au dysfonctionnement des muscles de clôture de l'urètre 7, des muscles du périnée, et des ligaments maintenant les organes de la cavité abdominale en position.

En effet, ces structures anatomiques, lorsqu'elles sont fonctionnelles, permettent de supporter une augmentation de la pression abdominale, due à une situation d'effort, comme un accès de toux, de rire, d'éternuements, ou un exercice physique.

En cas de dysfonctionnement des muscles de clôture de l'urètre 7, des muscles du périnée et des ligaments soutenant les organes de la cavité abdominale, la pression augmente dans la cavité abdominale suite notamment à un effort, cette pression se répercute sur la vessie 8 et les fuites d'urine peuvent survenir.

Afin de détecter le point de départ de l'augmentation de la pression intra-abdominale dans le temps, la sonde 1 est reliée à deux électrodes 15 électromyographiques disposées sur l'abdomen 16 du patient ou de la patiente. Ces électrodes 15 électromyographiques sont adaptées à enregistrer l'activité électromyographique abdominale qui est à l'origine de l'augmentation de la pression intra-abdominale.

De plus, dans l'objectif de détecter, suite à une augmentation intra-abdominale, le moment où se produit la fuite d'urine, l'embase 5 ex-vivo comporte au moins un détecteur d'urine 17 adapté à être mis en contact avec l'orifice urétral 70, le méat urinaire. Dans l'exemple illustré à la figure 2, le détecteur d'urine 17 est disposé au niveau de la partie antérieure de la vulve de la patiente.

Selon une caractéristique additionnelle non illustrée de l'invention, pour des raisons d'hygiène lors de son insertion au sein d'une cavité corporelle, la sonde de diagnostic 1 est équipée d'une enveloppe à usage unique qui recouvre le corps endocavitaire 4. L'enveloppe peut être réalisée en matériau polymérique biocompatible, par exemple en silicone.

Afin d'accroître le maintien de la sonde de diagnostic 1 au sein de l'endocavité lors de l'examen, l'enveloppe peut comprendre, à sa surface extérieure, un moyen de maintien coopérant avec les parois 3 de l'endocavité de manière à assurer le maintien de la sonde 1 en position lors de l'examen. Ces moyens de maintien peuvent être formés par une colle organique temporaire, ou bien une structure particulière de la surface extérieure de l'enveloppe qui aurait pour effet d'augmenter le coefficient de frottement de la surface extérieure de l'enveloppe avec la paroi 3 de l'endocavité.

Avantageusement, les caractéristiques de la sonde permettent de mesurer, en temps réel, lorsque la patiente est en situation d'effort, le point de départ de l'augmentation de la pression intra-abdominale définissant l'origine des mesures selon le temps, les pressions antéro-postérieures qui sont appliquées à l'endocavité, la déformation et le déplacement des organes entourant l'endocavité, la déformation et le déplacement des muscles du périnée, et le moment où la fuite urinaire se produit.

Tous ces paramètres permettent de mesurer le temps de réponse du réflexe myotatique de l'ensemble des structures par rapport à l'origine de l'augmentation de la pression intra-abdominale. Il est ainsi possible d'avoir des renseignements sur la qualité du réflexe myotatique de ces structures anatomiques et notamment des muscles pubo-vaginaux, pubo-rectaux, des sphincters urétraux et anaux, et des muscles du périnée.

De plus, la sonde de diagnostic 1 selon l'invention coopère avec des moyens de traitement des données qui sont adaptés à tracer les courbes d'accélération et d'amplitude de déformation de la paroi 3 endocavitaire dans les trois axes (X, Y et Z), c'est-à-dire, des courbes tridimensionnelles ou « 3D ». Chaque moyen de mesure 12 de déplacement de la sonde 1 génère des données qui permettent de tracer une courbe d'accélération et d'amplitude de mouvement 3D spécifique de la zone de la paroi 3 de l'endocavité avec laquelle il est placé en contact.

Ces courbes vont également permettre de déterminer quelle est l'amplitude du déplacement des structures anatomiques au niveau de chaque moyen de mesure 12, et à quel niveau d'accélération se produit le réflexe myotatique de ces structures anatomiques. Il est ainsi possible de suivre l'évolution de la déformation et du déplacement de ces structures anatomiques selon une échelle de temps prenant pour origine, le point de départ de l'augmentation de la pression intra-abdominale enregistré par les électrodes 15 électromyographiques.

Les moyens des traitements des données sont également adaptés à corréler les courbes de déplacement et de déformation, avec les données de pression antéro-postérieure, relevées par les capteurs 12 de pression, et qui s'exercent sur les parois 3 de la cavité corporelle.

De plus, en intégrant tous les paramètres mesurés par la sonde de diagnostic 1, les moyens de traitement des données sont adaptés à modéliser une vision tridimensionnelle dynamique du déplacement et de la déformation de l'endocavité, depuis l'origine de l'augmentation de la pression intra-abdominale enregistrée par les électrodes 15 électromyographiques. Cette modélisation permet de visualiser alors la déformation et le déplacement de l'endocavité ainsi que la déformation et le déplacement des muscles du périnée.

Ainsi, il est possible de déterminer précisément, à quel moment le réflexe myotatique de chaque structure anatomique se produit, à quel moment la fuite urinaire ou fécale se produit, à quel niveau de résistance la fuite urinaire se produit et quelle structure anatomique est responsable de cette fuite urinaire ou fécale et donc quelle structure anatomique est responsable de l'incontinence.

L'invention permet donc de réaliser des mesures précises du comportement des structures anatomiques liées au mécanisme de la continence, de manière à établir le diagnostic des dysfonctionnements affectant ces structures anatomiques responsables de l'incontinence urinaire ou fécale.

La patiente est ensuite orientée vers un traitement et/ou une rééducation adaptée(s), de manière très précise, grâce au diagnostic qui a été obtenu au moyen de la présente sonde de diagnostic 1.

## Revendications

1. Sonde de diagnostic (1) conçue apte à être introduite dans une endocavité corporelle, vaginale (2) ou rectale, du corps humain et à permettre, en réponse à une augmentation de pression intra-abdominale, au moins la mesure du déplacement des parois (3) de ladite endocavité et des organes qui l'entourent ainsi que la mesure du déplacement et de la résistance passive et active d'au moins un muscle du périnée, la sonde de diagnostic (1) comportant une embase (5) ex-vivo configurée pour être disposée à l'ouverture de l'endocavité, et reliée à un corps endocavitaire (4) équipé d'au moins deux capteurs (11) de pression disposés dans un même plan, et agissant soit dans une même direction, soit dans deux directions opposées, le corps endocavitaire (4) étant conçu apte à être déformable sans opposer de résistance et à épouser les parois (3) de ladite endocavité, la sonde de diagnostic (1) comportant :
- au moins un moyen de mesure (12, 120, 121, 122, 123) du déplacement de la paroi (3) endocavitaire dans les trois axes X, Y et Z, ledit au moins un moyen de mesure (12, 120, 121, 122, 123) du déplacement de la paroi (3) endocavitaire étant disposé sur le corps endocavitaire (4) ; et
- un moyen de mesure (12, 124) du déplacement des muscles du périnée selon trois axes X, Y, Z, ledit moyen de mesure (12, 124) du déplacement des muscles du périnée étant disposé sur l'embase (5) ;
ledit au moins un moyen de mesure (12) du déplacement de la paroi (3) endocavitaire étant relié à au moins un élément de référence spatial X, Y , Z, et ledit moyen de mesure (12) du déplacement des muscles du périnée étant relié à un élément de référence spatial X, Y, Z.

2. Sonde de diagnostic (1) selon la revendication 1, **caractérisée en ce qu'**elle est configurée apte à coopérer avec des moyens d'analyse des mesures de pression/déplacement effectuées et de reconstitution d'une vision tridimensionnelle dynamique du déplacement de ladite endocavité.

3. Sonde de diagnostic (1) selon l'une des revendications 1 à 2, **caractérisée en ce que** le corps endocavitaire (4) est formé par une bande souple (40) pliée en deux de manière à épouser, dans un plan sagittal, les parois (3) de ladite endocavité, le corps endocavitaire (4) comportant deux extrémités (44, 45) chacune reliée à l'embase (5) ex vivo de la sonde de diagnostic (1), l'embase (5) ex vivo comportant un moyen adhésif (52) biocompatible pour le renforcement du maintien en position de ladite sonde (1).

4. Sonde de diagnostic (1) selon la revendication 3, **caractérisée en ce que** l'embase (5) ex vivo comporte au moins un détecteur d'urine (17).

5. Sonde de diagnostic (1) selon l'une des revendications 1 à 4, **caractérisée en ce que** le moyen de mesure (12) du déplacement de la paroi (3) endocavitaire consiste en une centrale inertielle.

6. Sonde de diagnostic (1) selon l'une des revendications 1 à 5, **caractérisée en ce que** l'élément de référence spatial X,Y,Z comporte au moins deux moyens de mesure (124, 125) du déplacement du centre tendineux (13) du périnée et du sacrum (14) .

7. Sonde de diagnostic (1) selon l'une des revendications 1 à 6, **caractérisée en ce qu'**elle coopère avec au moins deux électrodes (15) de mesure d'un signal électro-myographique configurées pour être disposées sur l'abdomen (16) d'un ou d'une patient(e) de manière à enregistrer l'activité électromyographique abdominale à l'origine des pressions intra-abdominales.

8. Sonde de diagnostic (1) selon l'une des revendications 1 à 7, **caractérisée en ce que** le corps endocavitaire (4) comporte quatre moyens de mesure (120, 121, 122, 123) du déplacement de la paroi (3) endocavitaire, les moyens de mesure (120, 121, 122, 123) sont disposés à différents niveaux de profondeur lorsque la sonde de diagnostic (1) est insérée dans une endocavité.

9. Sonde de diagnostic (1) selon l'une des revendications 1 à 8, **caractérisée en ce que** le corps endocavitaire (4) comporte six capteurs (11, 110, 111, 112, 113, 114, 115) de pression.

10. Sonde diagnostic (1) selon la revendication 9, **caractérisée en ce que** les capteurs (11, 110, 111, 112, 113, 114, 115) de pression sont répartis deux à deux par paire, chaque paire de capteurs (11, 110, 111, 112, 113, 114, 115) de pression étant disposée à différents niveaux sur le corps endocavitaire (4).

11. Sonde de diagnostic (1) selon la revendication 10, **caractérisée en ce que** chaque paire de capteurs (11, 110, 111, 112, 113, 114, 115) de pression comporte un capteur de pression sur la partie antérieure (42) du corps endocavitaire (4) et un capteur de pression sur la partie postérieure (43) du corps endocavitaire (4).

12. Sonde de diagnostic (1) selon la revendication 11, **caractérisée en ce que** trois des moyens de mesure de déplacement (120, 121, 122) de la paroi endocavitaire sont disposés sur la partie antérieure (42) du corps endocavitaire (4), chacun respectivement à proximité immédiate d'un capteur (110, 112, 114) de pression placé sur la partie antérieure du corps endocavitaire et correspondant à une des paires de capteurs de pression.

13. Sonde de diagnostic (1) selon l'une des revendications 1 à 12, **caractérisée en ce qu'**elle coopère avec une enveloppe en matériau polymérique biocompatible recouvrant le corps endocavitaire (4).

## Patentansprüche

1. Diagnostiksonde (1), geeignet konzipiert, um in eine körperliche Endokavität, vaginal (2) oder rektal, des menschlichen Körpers eingeführt zu werden und als Reaktion auf eine Erhöhung des intraabdominalen Drucks mindestens die Messung der Verschiebung der Wände (3) der Endokavität und der Organe, die sie umgeben, sowie die Messung der Verschiebung und des passiven und aktiven Widerstands von mindestens einem Dammmuskel zu gestatten, wobei die Diagnostiksonde (1) eine Ex-vivo-Basis (5) umfasst, konfiguriert, um an der Öffnung der Endokavität angeordnet und mit einem endokavitären Körper (4), ausgerüstet mit mindestens zwei Drucksensoren (11), die in einer gleichen Ebene angeordnet sind und entweder in einer gleichen Richtung oder in zwei entgegengesetzten Richtungen wirken, verbunden zu werden, wobei der endokavitäre Körper (4) geeignet konzipiert ist, um verformbar zu sein, ohne Widerstand zu leisten, und sich an die Wände (3) der Endokavität anzuschmiegen, wobei die Diagnostiksonde (1) umfasst:
- mindestens ein Messmittel (12, 120, 121, 122, 123) der Verschiebung der endokavitären Wand (3) in den drei Achsen X, Y und Z, wobei mindestens ein Messmittel (12, 120, 121, 122, 123) der Verschiebung der endokavitären Wand (3) auf dem endokavitären Körper (4) angeordnet ist; und
- ein Messmittel (12, 124) der Verschiebung der Dammmuskeln gemäß drei Achsen X, Y, Z, wobei das Messmittel (12, 124) der Verschiebung der Dammmuskeln auf der Basis (5) angeordnet ist;
wobei mindestens ein Messmittel (12) der Verschiebung der endokavitären Wand (3) mit mindestens einem räumlichen Referenzelement X, Y, Z verbunden ist und wobei das Messmittel (12) der Verschiebung der Dammmuskeln mit einem räumlichen Referenzelement X, Y, Z verbunden ist.

2. Diagnostiksonde (1) nach Anspruch 1, **dadurch gekennzeichnet, dass** sie geeignet konfiguriert ist, um mit Analysemitteln der bewirkten Druck-/Verschiebungsmessungen und Rekonstitutionsmitteln einer dreidimensionalen dynamischen Ansicht der Verschiebung der Endokavität zusammenzuwirken.

3. Diagnostiksonde (1) nach einem der Ansprüche 1 bis 2,
**dadurch gekennzeichnet, dass** der endokavitäre Körper (4) durch ein in zwei gefaltetes weiches Band (40) derart gebildet ist, um sich in einer sagittalen Ebene an die Wände (3) der Endokavität anzuschmiegen, wobei der endokavitäre Körper (4) zwei Enden (44, 45) umfasst, von denen jedes mit der Ex-vivo-Basis (5) der Diagnostiksonde (1) verbunden ist, wobei die Ex-vivo-Basis (5) ein biokompatibles Haftmittel (52) für die Verstärkung des Haltens der Sonde (1) in Position umfasst.

4. Diagnostiksonde (1) nach Anspruch 3, **dadurch gekennzeichnet, dass** die Ex-vivo-Basis (5) mindestens einen Urindetektor (17) umfasst.

5. Diagnostiksonde (1) nach einem der Ansprüche 1 bis 4,
**dadurch gekennzeichnet, dass** das Messmittel (12) der Verschiebung der endokavitären Wand (3) aus einer Trägheitsmesseinheit besteht.

6. Diagnostiksonde (1) nach einem der Ansprüche 1 bis 5,
**dadurch gekennzeichnet, dass** das räumliche Referenzelement X, Y, Z mindestens zwei Messmittel (124, 125) der Verschiebung des Sehnenmittelpunkts (13) des Damms und des Kreuzbeins (14) umfasst.

7. Diagnostiksonde (1) nach einem der Ansprüche 1 bis 6,
**dadurch gekennzeichnet, dass** sie mit mindestens zwei Messelektroden (15) eines elektromyographischen Signals zusammenwirkt, konfiguriert, um auf dem Abdomen (16) eines Patienten oder einer Patientin derart angeordnet zu werden, um die abdominale elektromyographische Aktivität an dem Ursprung der intraabdominalen Drücke zu registrieren.

8. Diagnostiksonde (1) nach einem der Ansprüche 1 bis 7,
**dadurch gekennzeichnet, dass** der endokavitäre Körper (4) vier Messmittel (120, 121, 122, 123) der Verschiebung der endokavitären Wand (3) umfasst, wobei die Messmittel (120, 121, 122, 123) an unterschiedlichen Tiefenniveaus angeordnet werden, wenn die Diagnostiksonde (1) in eine Endokavität eingefügt wird.

9. Diagnostiksonde (1) nach einem der Ansprüche 1 bis 8,
**dadurch gekennzeichnet, dass** der endokavitäre Körper (4) sechs Drucksensoren (11, 110, 111, 112, 113, 114, 115) umfasst.

10. Diagnostiksonde (1) nach Anspruch 9, **dadurch gekennzeichnet, dass** die Drucksensoren (11, 110, 111, 112, 113, 114, 115) zwei und zwei pro Paar verteilt sind, wobei jedes Paar von Drucksensoren (11, 110, 111, 112, 113, 114, 115) an unterschiedlichen Niveaus auf dem endokavitären Körper (4) angeordnet ist.

11. Diagnostiksonde (1) nach Anspruch 10, **dadurch gekennzeichnet, dass** jedes Paar von Drucksensoren (11, 110, 111, 112, 113, 114, 115) einen Drucksensor auf dem vorderen Teil (42) des endokavitären Körpers (4) und einen Drucksensor auf dem hinteren Teil (43) des endokavitären Körpers (4) umfasst.

12. Diagnostiksonde (1) nach Anspruch 11, **dadurch gekennzeichnet, dass** drei der Messmittel der Verschiebung (120, 121, 122) der endokavitären Wand auf dem vorderen Teil (42) des endokavitären Körpers (4) angeordnet sind, jedes jeweils in unmittelbarer Nähe eines Drucksensors (110, 112, 114), der auf dem vorderen Teil des endokavitären Körpers platziert ist und einem der Paare von Drucksensoren entspricht.

13. Diagnostiksonde (1) nach einem der Ansprüche 1 bis 12,
**dadurch gekennzeichnet, dass** sie mit einer Umhüllung aus biokompatiblem Polymermaterial zusammenwirkt, die den endokavitären Körper (4) bedeckt.

## Claims

1. A diagnostic probe (1) designed to be introduced into a bodily, vaginal (2) or rectal endocavity of the human body and to allow, in response to an increase in intra-abdominal pressure, at least the measurement of the displacement of the walls (3) of said endocavity and of the organs which surround it as well as the measurement of the displacement and of the passive and active resistance of at least one muscle of the perineum, the diagnostic probe (1) comprising an ex-vivo base (5) configured to be arranged at the opening of the endocavity, and connected to an intracavitary body (4) equipped with at least two pressure sensors (11) arranged in the same plane, and acting either in the same direction, or in two opposite directions, the intracavitary body (4) being designed to be deformable without opposing resistance and to conform to the walls (3) of said endocavity, the diagnostic probe (1) comprising:
- at least one means (12, 120, 121, 122, 123) for measuring the displacement of the intracavitary wall (3) in the three axes X, Y and Z, said at least one means (12, 120, 121, 122, 123) for measuring the displacement of the intracavitary wall (3) being arranged on the intracavitary body (4); and
- a means (12, 124) for measuring the displacement of the perineum muscles along three axes X, Y, Z, said means (12, 124) for measuring the displacement of the perineum muscles being arranged on the base (5);
said at least one means (12) for measuring the displacement of the intracavitary wall (3) being connected to at least one spatial reference element X, Y, Z, and said means (12) for measuring the displacement of the perineum muscles being connected to a spatial reference element X, Y, Z.

2. The diagnostic probe (1) according to claim 1, **characterized in that** it is configured to mate with means for analyzing the pressure/displacement measurements carried out and to reconstitute a dynamic three-dimensional vision of the movement of said endocavity.

3. The diagnostic probe (1) according to one of claims 1 to 2, **characterized in that** the intracavitary body (4) is formed by a flexible strip (40) folded in two so as to conform, in a sagittal plane, to the walls (3) of said endocavity, the intracavitary body (4) comprising two ends (44, 45) each connected to the ex vivo base (5) of the diagnostic probe (1), the ex vivo base (5) comprising a biocompatible adhesive means (52) for strengthening the holding of said probe (1) in position.

4. The diagnostic probe (1) according to claim 3, **characterized in that** the ex vivo base (5) comprises at least one urine detector (17).

5. The diagnostic probe (1) according to one of claims 1 to 4, **characterized in that** the means (12) for measuring the displacement of the intracavitary wall (3) consists of an inertial unit.

6. The diagnostic probe (1) according to one of claims 1 to 5, **characterized in that** the spatial reference element X, Y, Z comprises at least two means (124, 125) for measuring the displacement of the muscular centre of the perineum (13) and of the sacrum (14).

7. The diagnostic probe (1) according to one of claims 1 to 6, **characterized in that** it mates with at least two electrodes (15) for measuring an electromyographic signal configured to be arranged on the abdomen (16) of a patient so as to record the abdominal electromyographic activity from which the intra-abdominal pressures originate.

8. The diagnostic probe (1) according to one of claims 1 to 7, **characterized in that** the intracavitary body (4) comprises four means (120, 121, 122, 123) for measuring the displacement of the intracavitary wall (3), the measuring means (120, 121, 122, 123) being arranged at different depth levels when the diagnostic probe (1) is inserted into an endocavity.

9. The diagnostic probe (1) according to one of claims 1 to 8, **characterized in that** the intracavitary body (4) comprises six pressure sensors (11, 110, 111, 112, 113, 114, 115).

10. The diagnostic probe (1) according to claim 9, **characterized in that** the pressure sensors (11, 110, 111, 112, 113, 114, 115) are distributed in pairs, each pair of pressure sensors (11, 110, 111, 112, 113, 114, 115) being arranged at different levels on the intracavitary body (4).

11. The diagnostic probe (1) according to claim 10, **characterized in that** each pair of pressure sensors (11, 110, 111, 112, 113, 114, 115) comprises a pressure sensor on the anterior portion (42) of the intracavitary body (4) and a pressure sensor on the posterior portion (43) of the intracavitary body (4).

12. The diagnostic probe (1) according to claim 11, **characterized in that** three of the means (120, 121, 122) for measuring the displacement of the intracavitary wall are arranged on the anterior portion (42) of the intracavitary body (4), each respectively in the immediate vicinity of a pressure sensor (110, 112, 114) placed on the anterior portion of the intracavitary body and corresponding to one of the pairs of pressure sensors.

13. The diagnostic probe (1) according to one of claims 1 to 12, **characterized in that** it mates with a shell made of biocompatible polymeric material covering the intracavitary body (4).
